# EUROPEAN PATENT APPLICATION

(11) **EP 4 692 368 A1**
(43) Date of publication of application: **11.02.2026**
(21) Application number: 24382881.1
(22) Date of filing: 05.08.2024
(51) Int. Cl.: C12Q 1/6825, G01N 33/00

(54) **MULTIPLEXED BIODETECTION SYSTEM**

(71) Applicant: Mecwins, S.A., 28760 Tres Cantos Madrid (ES)
(72) Inventor: RODRÍGUEZ MARIBLANCA, Isabel, 28760 TRES CANTOS (Madrid) (ES); PINI, Valerio, 28760 TRES CANTOS (Madrid) (ES); CEBRIÁN HERNANDO, Virginia, 28760 TRES CANTOS (Madrid) (ES); THON, Andreas, 28760 TRES CANTOS (Madrid) (ES); AHUMADA HEREDERO, Jesús Óscar, 28760 TRES CANTOS (Madrid) (ES); TABRAUE CHÁVEZ, Mavys, 18016 GRANADA (ES); PERNAGALLO, Salvatore, 18016 GRANADA (ES); DÍAZ-MOCHÓN, Juan José, 18016 GRANADA (ES)
(74) Representative: Herrero & Asociados, S.L.

(57) **Abstract**

The invention relates to a multiplexed biodetection system arranged for simultaneously detecting from a liquid sample at least a target analyte of peptidic or proteic nature and at least a target analyte of nucleic acid nature such as a DNA or RNA molecule. The system comprises two basic elements, a substrate with a surface functionalized with streptavidin to anchor at least two different arrangement of elements having bound thereto a nanoparticle each of which has different plasmonic properties. Each of these at least two arrangement of elements allows to simultaneously detect at least a target analyte of peptidic or proteic nature and at least a target analyte of nucleic acid nature due to the different the plasmonic effect produced by each of the different nanoparticles used. The invention also refers to a multiplexed biodetection and to a biosensing platform based on the biodetection system of the invention.

## Description

### FIELD OF THE INVENTION

The present invention belongs to the field of biosensors. More particularly, the present invention relates to a multiplexed biodetection system arranged for simultaneously detecting from a liquid sample at least a target analyte of peptidic or proteic nature and at least a target analyte of nucleic acid nature such as a DNA or RNA molecule. The system comprises two basic elements, a substrate with a surface functionalized with streptavidin to anchor at least two different arrangements of elements having bound thereto a nanoparticle each of which has different plasmonic properties. Each of these at least two arrangements of elements allow to simultaneously detect at least a target analyte of peptidic or proteic nature and at least a target analyte of nucleic acid nature due to the different plasmonic effect produced by each of the different nanoparticles used.

The invention also relates to a biosensor incorporating such system, in addition to a method for a multiplexed and simultaneous detection and quantification of at least one target analyte of peptidic or proteic nature and at least one target analyte of nucleic acid nature in a sample using such system. Finally, the invention relates to a biosensing platform which can detect and distinguish the different plasmonic effects produced by the different nanoparticles in the system of the invention by image analysis that allows ultra-high sensitivity, as it can be achieved with digital single-particle counting.

### BACKGROUND OF THE INVENTION

Early diagnosis of disease is essential for effective therapeutic management, which is of paramount importance for patient prognosis and survival [1]. The use of promising biomarkers with high sensitivity and specificity is an important basis for the accurate diagnosis of early-stage disease [2, 3].

While biomedical diagnostics based on single analyte assays can only provide limited information, multiplexed biosensors have many advantages over single analyte assays because they can increase throughput and improve test efficiency [4].

The combination of protein and nucleic acid markers could be useful for the diagnosis of early-stage disease, improving the sensitivity and specificity of diagnosis [5, 6]. In addition, in the case of cancer, the combination of biomarkers can also provide information about the stage of the cancer as well as the response to therapy [7]. Despite their high value as gold standards, neither ELISA nor PCR can detect other types of targets, but only proteins and nucleic acids, respectively. Early technologies and platforms for the simultaneous detection of nucleic acids and proteins were based on complex and expensive instruments (multiparameter cell analysis system) [8] or the combination of nucleic acid detection (DNA array, PCR) and protein detection (protein array, ELISA) [6, 9-11]. However, the combination of ELISA (enzyme-linked immunosorbent assay) and PCR (polymerase chain reaction) is not the most suitable for the simultaneous detection of nucleic acids and proteins because they have different detection mechanisms and dynamic ranges. Combining ELISA and PCR requires separate assays for each type of analyte, which can increase the cost, time, and complexity of the analysis. In addition, PCR requires additional steps for nucleic acid extraction and amplification, which can increase the risk of error, contamination, and sample loss [12-20].

Therefore, there is growing interest in the development of platforms or systems that can simultaneously detect nucleic acids and proteins. Several types of platforms have been developed and tested, including microfluidic devices, biosensors, and microarrays [12-20]. The advantages of using this type of combined detection systems are [12-20]:
- Simultaneous detection of nucleic acids and proteins can provide a more complete picture of disease status and progression than either type of analysis alone.
- Combining nucleic acid and protein analysis may allow for more sensitive and specific detection of disease markers.
- Simultaneous detection may also be more efficient and cost-effective than performing separate nucleic acid and protein assays.
- Simultaneous detection allows for smaller sample volumes as everything is analysed in the same system.
- Time and cost reduction as only one single assay has to be performed.

In addition, these combined detection platforms must have several characteristics to be competitive [12-20]:
- The platform should be able to simultaneously detect both nucleic acids and proteins in the same sample.
- The platform should be sensitive enough to detect low levels of analytes in complex biological matrices.
- The platform should be specific enough to distinguish between different analytes and avoid cross-reactivity or interference from other components in the sample.
- The platform should be easy to use and easily adaptable to different applications and sample types.

The combined nucleic acid and protein detection platforms can detect various biomarkers, including viral nucleic acids and proteins for the detection of viral infections such as HIV and SARS-CoV-2, cancer biomarkers including microRNAs and cancer-specific proteins such as PSA (prostate specific antigen), specific genetic mutations associated with various diseases, immune system biomarkers such as cytokines, or neurological biomarkers such as tau protein or amyloid beta protein associated with neurodegenerative diseases such as Alzheimer's and Parkinson's disease [12-20].

Among nucleic acids, the detection of microRNAs has become increasingly important because microRNAs are small non-coding RNAs that play important roles in many biological processes, including development, differentiation, and disease progression [21]. RT-qPCR (reverse transcription quantitative polymerase chain reaction) is considered the gold standard for miRNA analysis, providing high sensitivity and specificity with a wide dynamic range [22]. However, the use of RT-qPCR for the direct detection of miRNAs in liquid biopsies without RNA extraction is characterized by lower sensitivity, precision, and reproducibility [23]. In addition, the complex design of structuring primers or the use of primers containing blocked nucleic acid bases increases the cost of the technique and implies a more complicated design, making its widespread use difficult [24]. Therefore, reproducible and robust quantification of miRNAs in liquid biopsies remains a challenge. For these reasons, the development of platforms for the combined detection of proteins and nucleic acids is essential for the detection of miRNAs.

In summary, the development of platforms that enable the combined detection of nucleic acids and proteins is essential.

The authors of the present invention have developed a multiplexed biodetection system that allows the simultaneous detection of nucleic acids and proteins of interest in pathological processes in biofluids. The advantages of this new technology are:
- Sensitivity: Between 10 (compared to NAT [nucleic acid test]) and 1000 (compared to ELISA) times more sensitive than currently available techniques, which translates into greater reliability of existing diagnostic tests and/or the possibility of accessing tests that do not currently exist.
- Speed: Faster than current detection techniques, providing greater ease of use and the ability to automate measurements.
- Small sample volume for analysis: Although the sensitivity of this technology is very high, the required sample volume is quite small (maybe as low as 10 microliters, depending on the dilution used).
- No extraction or purification of genetic material or enzymatic amplification of the genetic target of interest is required, resulting in a simpler methodology and a lower risk of sample contamination and material loss.
- High specificity: The system accurately differentiates between target molecules and other substances, ensuring that false positives are minimized. This precision is crucial for ensuring the reliability of diagnostics, especially in situations where similar pathogens may be present or when detecting trace amounts of a target substance.

### BRIEF DESCRIPTION OF THE FIGURES

**Figure 1****:** represents two variations or approaches for a duplex biodetection of proteic and nucleic acid targets. Approach A includes the combination of arrangement of elements (b.1) and (b.3) and approach B includes the combination of arrangement (b.1) and (b.2).
**Figure 2****:** two-dimensional histogram based on particle color and brightness obtained by AVAC image analyzer in which nanoparticles of different sizes are differentiated (100 nm and 150 nm).
**Figure 3****:** Flowchart representing the different software-driven operations carried out during image analysis resulting in counting and determination of the concentration of the different biomarkers in the samples.
**Figure 4****:** is a schematic representation of the biosensing platform according to the invention.
**Figure 5****:** represents the results of approach A in duplex. The results are represented in non-specific counts, specific counts and ratio (signal/background) of IL-6 and miR-122. In the case of IL-6 the specific counts or the signal have been tested at 150 pg/ml and in the case of miR-122 at 300 pM.
**Figure 6****:** represents the results of approach B in duplex. The results are represented in non-specific counts, specific counts and ratio (signal/background) of IL-6 and miR-122. In the case of IL-6 the specific counts or the signal have been tested at 150 pg/ml and in the case of miR-122 at 300 pM.
**Figure 7** results in calibration curves and sensitivity (LOQ) of the miR-122 detection with the different tested conditions with approach B, varying the GNPs and capture antibodies proportion: standard, increase in the GNPs with PNA-122 amount, increase in the anti-FITC amount and increase in both.
**Figure 8****:** results in calibration curves and sensitivity (LOQ) of the miR-122 detection with the different tested conditions with approach B, varying the GNPs and capture antibodies proportion: standard, increase in the GNPs with PNA-122 amount, increase in the anti-FITC amount and increase in both.

### DESCRIPTION OF THE INVENTION

The following definitions are provided to aid in the understanding and interpretation of the present invention:
**Biosensor:** An analytical device comprising a biological capture element (for example, an antibody, a receptor, a peptide, a protein, a Peptidic Nucleic Acid ( PNA) in close contact with a transducer of electrochemical signals, mechanical signals, optical signals, thermal signals, acoustic signals, or other physical signals which, in combination, allow chemical property analysis or target analyte detection or quantification.
**Multiplexed system:** it refers to a system that allows for the simultaneous detection of several target analytes from the same sample.
**Target analyte:** It is the molecule sought for detection from a sample by the system of the invention, preferably from a liquid sample. The target analyte to be detected by the system of the invention may be of peptidic or proteic nature, or of nucleic acid nature.
**Target analyte of peptidic nature:** it refers to a proteic or peptidic biomarker. As a non-limiting list, the target analyte of peptidic nature may be a viral protein (such as HIV and SARS-CoV-2 proteins), cancer biomarkers (such as PSA for prostate cancer), neurological biomarkers (such as tau protein or amyloid beta protein associated with neurodegenerative diseases), immune system biomarkers (such as cytokines), or any other type of molecule formed by a sequence of aminoacids.
**Target analyte of nucleic acid nature:** it refers to a nucleic acid biomarker, either RNA or DNA biomarkers. These can be, for instance, polynucleotides or oligonucleotides formed by a specific sequence of nucleotides having viral origin or expressed during development of a certain disease or condition such as for instance tumorigenesis or cancer development and evolution. A non-limiting list of possible target analytes of nucleic acid nature may be genes, SNPs, ESTs, mRNA, tARN, iRNA, rRNA, miRNAs, or mitochondrial DNA.
Sample: It refers to the biological material to be analyzed and allegedly containing the target analytes to be detected. It is preferably a fluid such as blood, serum, plasma, urine, saliva, bile, cerebrospinal fluid, semen, or vaginal discharge.
Dielectric material: A dielectric material is an electric insulator which can be polarized by an applied electric field. A non-limiting list of dielectric materials are silicon oxide, silicon, silicon nitride, silicon carbide, graphene, polymers, and hydrogels.
Functionalized surface or surface functionalization: A method or technique for introducing functional groups on a surface. The latter is used in biosensors for immobilizing a capture element on a surface, in the present invention, on the substrate surface. In the context of the present invention, the surface is functionalized with streptavidin that allows the anchorage of biotinylated recognition element.
Streptavidin: it is a tetrameric protein having an extraordinary affinity for biotin. In the present invention, it is used for functionalizing the surface of the substrate of dielectric material. The streptavidin functionalization allows for anchoring the biotinylated capture elements through the strong streptavidin/biotin binding.
**Arrangement of elements:** It refers to the design, structural disposition and interrelation of the different elements sought to detect the target analyte to then get anchored to the streptavidin functionalized surface of the substrate of dielectric material. The invention comprises three different arrangements of elements, referred to as (b. 1), (b.2) and (b.3) in claim 1. Arrangement (b.1) is sought for detection of target analytes of peptic or proteic nature and arrangements (b.2) and (b.3) are both sought for detection of target analytes of nucleic acid nature. The system of the invention comprises the presence of at least arrangement (b.1) and at least one of arrangements (b.2) and (b.3).
**Biotinylated capture element:** it is the element of the system which gets anchored to the functionalized surface of the substrate of dielectric material through the streptadvidin/biotin binding. The biotinylated capture element is formed by the capture element itself that can recognize and specifically bind to one of the target analytes which has bound thereto the biotin molecule. In the context of the invention, the biotinylated recognition element is preferably selected from a biotinylated antibody or a biotinylated nucleobase. Those embodiments where the capture element is a biotinylated antibody may be sought for detection of target analytes of either peptidic nature or of nucleic acid nature. In contrast, those embodiments where the capture element is a biotinylated nucleobase are sought only for arrangements suitable for detecting target analytes of nucleic acid nature.
**Fluorophore-modified nucleobase:** It is a nucleobase directed to fill the single nucleotide gap between the duplex PNA/target analyte, and after reduction thereof gets irreversibly fixed to said duplex. After fixing of the PNA/target analyte, a fluorophore-modified duplex is obtained that can be captured by the biotinylated capture element which in turn is anchored to the surface of the dielectric substrate functionalized with streptavidin. The fluorophore-modified nucleobase is used in arrangement (b.2) in the system of the invention.
**Biotinylated nucleobase:** It is a nucleobase directed to fill the single nucleotide gap between the duplex PNA/target analyte and after reduction thereof gets irreversibly fixed to said duplex. After fixing of the PNA/target analyte, a biotinylated duplex is obtained that can be anchored to the surface of the dielectric substrate functionalized with streptavidin. The biotinylated nucleobase is used in arrangement (b.3) in the system of the invention.
**Detection element:** It is the element of the system bound to the nanoparticle which can specifically bind to one of the target analytes. The detection element thanks to the nanoparticle bound thereto allows the target analyte detection when it is present in the sample.
**Plasmonic effect or Localized Surface Plasmon Resonance (LSPR):** it refers to the unique optical property exhibited by certain metallic nanoparticles when exposed to electromagnetic radiation. The LSPR phenomenon is a result of collective electron oscillations within the nanoparticle, resonating at specific wavelengths. This resonance can lead to pronounced light absorption and scattering. This effect is distinct from surface plasmon waves, which is a different phenomenon.
**Nanoparticles with different plasmonic properties:** As used herein, the term refers to a specific subset of nanoparticles tailored for attachment to diverse detection elements. The purpose is to facilitate a distinct and discernible optical response for each target analyte upon detection. It is imperative that the plasmonic effect of the nanoparticle associated with one target analyte differs discernibly from the plasmonic effect of nanoparticles associated with another target analyte. Several factors may influence these distinct plasmonic properties:
   - Material Composition: Different nanoparticle materials inherently demonstrate distinct plasmonic characteristics.
   - Dimensional Parameters: Altering the size, even within nanoparticles of an identical material composition, can lead to variations in plasmonic responses.
   - Geometric Configuration: Nanoparticles can be fabricated in various shapes, each contributing to its unique plasmonic behaviour. Exemplary shapes include, but are not limited to, nanospheres, nanorods, pointed nanorods, nanoshells, nanocages/frames, hollow nanospheres, tetrahedra, octahedra, cubes, icosahedra, rhombic dodecahedra, concave nanocubes, tetrahexahedra, obtuse triangular bipyramids, trisohectahedra, and nanoprisms.
      Layered Structures: The introduction of core/shell designs or analogous multilayered configurations can further modulate inherent plasmonic properties.
**Antibody:** A Y-shaped protein (immunoglobulin) on the surface of B lymphocytes which is secreted into the blood or lymph in response to an antigenic stimulus, such as a bacterium, virus, parasite, or transplanted organ, and which neutralizes the antigen by binding specifically to it. The antibody-antigen pair formation can be detected by several methods.
**Detection:** It is the action of identifying the presence or absence of the target analyte in the sample.
**Quantification:** It is the action of determining the concentration of a target analyte in the sample.
**Electromagnetic radiation:** is a fundamental phenomenon of electromagnetism, behaving like waves propagating through space and carrying radiant energy. An electromagnetic wave has both electric and magnetic field components which oscillate in a fixed ratio with respect to one another, are perpendicular to one another, and perpendicular to the energy and wave propagation direction.
**Light scattering:** it refers to the phenomenon by which small portions of incident electromagnetic waves are dispersed in various directions due to interactions with a material or medium. While some of the incident light may reflect off a surface in a predictable manner, known as specular reflection, light scattering is characterized by its non-uniform, diffuse reflection or transmission. This diffuse component is not confined to a single direction, but rather spreads over a range of angles. The primary causes of light scattering can be attributed to surface irregularities (non-zero surface roughness) or the presence of particles within the medium or deposited on the surface.
**Calibration curve:** it refers to a pattern obtained by measuring a certain target analyte at different known concentrations. The calibration curve allows to correlate the count of nanoparticles with predetermined analyte concentrations, facilitating precise quantification of the analyte in the sample.
**Negative Control:** In the context of this invention, the negative control is defined by the introduction of the detection elements having bound thereto nanoparticles with plasmonic properties in the absence of analyte. Due to the inherent limitations of the system, it is not feasible to directly discern whether a specific nanoparticle has a target analyte attached or not. To ascertain this, a calibration curve is constructed by employing the detection elements having bound thereto nanoparticles with varying concentrations of the different target analytes. The negative control, crucial for establishing the baseline of the system, is determined using functionalized nanoparticles in the absence of analyte. This control helps in understanding the system's response in the absence of the target analyte and aids in identifying potential non-specific signals.

### Multiplexed biodetection system

A first object of the invention relates to a multiplexed biodetection system arranged to simultaneously detect at least one target analyte of peptidic nature and at least one target analyte of nucleic acid nature from a sample comprising:
a) a substrate of dielectric material having a surface functionalized with streptavidin capable of anchoring biotinylated elements; and
b) at least two of the following arrangement of elements:
   (b.1) a biotinylated capture element capable of specifically binding to a target analyte of peptidic nature and a detection element having bound thereto a nanoparticle with plasmonic properties which is capable of specially binding to the target analyte in a sandwich type arrangement;
   (b.2) a biotinylated capture element capable of specifically binding to a fluorophore of a fluorophore-modified nucleobase which can only be detected when specific hybridization between a target analyte of nucleic acid nature and a of fully complementary except-for-a-single-nucleobase PNA takes place, wherein the PNA, acting as detection element, has a nanoparticle with plasmonic properties bound thereto and wherein the detection occurs after the fluorophore-modified nucleobase fills the single nucleotide gap between the duplex PNA/target analyte and whereby after a reduction reaction said duplex is irreversibly fixed;
   (b.3) a biotinylated nucleobase as capture element which can only be detected when specific hybridization between a target analyte of nucleic acid nature and a of fully complementary except-for-a-single-nucleobase PNA takes place, wherein the PNA, acting as detection element, has a nanoparticle with plasmonic properties bound thereto and wherein the detection occurs after the biotinylated nucleobase fills the single nucleotide gap between the duplex PNA/target analyte and whereby after a reduction reaction said duplex is irreversibly fixed;
with the proviso that the system comprises at least arrangement (b.1) and at least one of arrangements (b.2) or (b.3) and with the proviso that the nanoparticles used in detection of each target analyte have different plasmonic properties.

This first object of the invention will indistinctly be referred along the following lines as "multiplexed biodetection system of the invention", "biodetection system of the invention" or simply "the system of the invention".

The system of the invention is suitable for the simultaneous detection and/or quantification of at least two different types of target analyte of different nature. This means that the system may be used and arranged to simultaneously detect from the same sample at least one target analyte of proteic or peptidic nature and a target analyte of nucleic acid nature. The possibility of detecting proteic and nucleic acid markers is useful in diagnosis, especially in early-stage disease as it provides a more complete picture of disease status and progression. The combined detection also allows for the improvement of sensitivity and specificity of diagnosis and/or prognosis.

The system of the present invention is based on the use of capture elements and detection elements. This double check in detection also greatly improves the sensitivity and specificity of the system. Notwithstanding, the most disruptive aspect of the system of the invention apart from the simultaneous and direct detection of peptidic markers and nucleic acid markers from the same sample is derived from the use of different nanoparticles with different plasmonic effects as detection tags. Plasmonic nanoparticles allow ultra-low limits of detection of each target analyte as well as detection and/or quantification by optical means.

The nanoparticles useful in the system of the invention must be capable of producing a plasmonic effect. In this sense, the nanoparticles must have certain dimensions and being made of certain materials.

The plasmonic resonance peak is highly dependent on the nanoparticle size and so nanoparticles must be in the nanometric scale and more preferably each of the nanoparticles used must have at least one of its dimensions with a size of 2 nm to 300 nm, preferably 5 nm to 200 nm. This means that the longer diameter of the nanoparticle must be within the aforementioned range.

Regarding the material, any type of nanoparticle with plasmonic properties can be used. Therefore, the nanoparticle can be, for example, a gold nanoparticle, a silver nanoparticle, or a nanoparticle of plasmonic metamaterial such as, but not limited to, titanium nitride and non-stoichiometric oxides such as non-stoichiometric vanadium, titanium, and aluminum oxides. Another possibility is the utilization of multi-layered particles, which can also exhibit unique plasmonic properties due to their core/shell structures. A preferred embodiment is represented by gold nanoparticles.

Furthermore, the nanoparticle can adopt a plurality of forms or structures, such as for example, nanospheres, nanorods, pointed nanorods, nanoshells, nanocages/frames, hollow nanospheres, tetrahedra, octahedra, cubes, icosahedra, rhombic dodecahedra, concave nanocubes, tetrahexahedra, obtuse triangular bipyramids, trisohectahedra and nanoprisms.

The variation of these three parameters of the nanoparticles, i.e. material, dimensions, and shape, allows for nanoparticles with different plasmonic properties each of which can be used as a labeling or tag associated with detection of a specific target analyte. The differences of the plasmonic effect are such that they can be optically identified such as, for instance, by a different color and intensity of the light scattered by the plasmonic nanoparticle when irradiated with an electromagnetic radiation source.

The substrate must be a dielectric material such that the localized plasmon resonance phenomenon can take place and potentially be enhanced. Any dielectric material in the electromagnetic spectral range of interest is suitable in the system of the invention. In a particular embodiment, the dielectric material includes silicon oxide, silicon, silicon nitride, silicon carbide, graphene, polymers such as SU8, COP, COC, SMMA, PMMA, and hydrogels like mixtures of PEG and PLA or of DEXTRAN and PEG. The most preferred dielectric materials are silicon, silicon oxide, COP, and COC.

Another essential aspect of invention is the arrangement of the different elements forming the system of the invention. The substrate is prepared to anchor the different capture elements. For the anchorage of the different capture elements, the surface of the substrate of dielectric material is functionalized with streptavidin. Streptavidin functionalization may be carried out by any well-known method in the art such as the use of glutaraldehyde as a linker [25].

In turn, the different capture elements are all biotinylated. The biotinylation of the capture elements must be done by any well-known method in the art in a position of the capture element not affecting its capacity to bind the element to be captured. Biotionylation of the capture elements can be carried out by any well-known method in the art such as engineering the capture element or biotinylation of the capture element amino groups with a sulfo-NHS biotin [26, 27]. Biotinylation of the capture elements allows their anchorage to the functionalized substrate through the strong non-covalent binding reaction between streptavidin and biotin. The use of the pair streptavidin/biotin allows that anchorage can be equally performed for any capture element irrespective of whether the recognition element is directed to ultimately recognize a target analyte of peptidic nature or a target analyte of nucleic acid nature.

The capture elements are molecules that have the capacity to either specifically bind a target analyte or that are part of an arrangement directed to ultimately capture a target analyte. In this sense, the system of the invention allows for different arrangements of elements depending on the type of capture element, the type of detection element and also in the nature of the target analyte to be detected.

For instance, when a proteic or peptidic target analyte is to be detected there is only one single arrangement possible, referred to above as (b.1). In this case the capture element is a biotinylated capture element, preferably a biotinylated antibody, capable of directly and specifically binding and capturing the peptidic or proteic target analyte. Additionally, for such an arrangement, the detection element is also preferably an antibody capable of specifically binding to the peptidic or proteic target analyte but having bound thereto a nanoparticle with plasmonic properties. This allows for the detection in a sandwich-type arrangement after irradiation with an electromagnetic radiation source producing the plasmonic effect in the nanoparticles.

When the target analyte to be detected is of nucleic acid nature, then there are two possible arrangements or designs for target detection.
1) In a first arrangement, referred to above as (b.2), the capture element is also a biotinylated capture element, preferably an antibody capable of specifically binding and capturing a molecule of a modified nucleobase. The molecule modifying said nucleobase is a fluorophore, more preferably it is fluoresceine isothiacyanate (FITC). In this arrangement, the detection element is a PNA molecule having a nanoparticle with plasmonic properties bound thereto and being fully complementary to the sequence of the target nucleic acid except for one nucleobase that must be filled after a reaction with the fluorophore-modified nucleobase if hybridization with the target sequence has previously taken place. For the detection to take place, a reaction between the duplex PNA/target nucleic acid in the presence of the above-mentioned fluorophore-modified nucleobase and a reducing agent must be carried out, preferably in the presence of a buffer at pH 5-7, preferably 6. After, this reaction the fluorophore-modified nucleobase will form a stable and irreversibly fixed product, i.e. a duplex PNA/target analyte having a fluorophore as part of the duplex structure and having a nanoparticle with plasmonic properties bound thereto. Now the fluorophore-modified PNA/target analyte duplex can be captured through the fluorophore, by the biotinylated capture element (i.e. an antibody). Detection can be carried out by irradiation with an electromagnetic radiation source producing the plasmonic effect in the nanoparticles. In this arrangement, the advantage of detecting nucleic acids with a biotinylated antibody is that the methodology is shorter and simpler because it is more compatible with the detection of protein biomarkers. In this case, it can be started from surfaces with streptavidin on which biotinylated antibodies have previously been immobilized, with the capture antibody for the protein biomarker and with the capture antibody for the fluorophore present in the capture of the nucleic acid biomarker.
2) In a second arrangement referred above as (b.3), the capture element is a biotinylated nucleobase which, as in the previous arrangement, has the purpose of filling the one single nucleotide gap in the duplex between the PNA, acting as detection element, and the target analyte (the target sequence) and irreversibly fix said duplex after a suitable reaction. In this arrangement, the detection element is the PNA having bound thereto a nanoparticle with plasmonic properties and having a sequence fully complementary to the target nucleic acid except for one single nucleobase. Detection is based on a similar technology as explained in the previous arrangement. Once the duplex PNA/target nucleic has hybridized a reaction between said duplex with biotinylated nucleobase in the presence of a reducing agent, preferably in the presence of a buffer at pH 5-7, preferably 6, must be carried out. The resulting product is a stable biotinylated PNA/target analyte duplex having bound thereto a nanoparticle with plasmonic properties. In this sense, if the target analyte is present in the sample analyzed, the resulting reaction product can be anchored to the functionalized substrate of dielectric material by the streptavidin/biotin binding and will be detected by the plasmonic effect produced by the nanoparticle when irradiated with an electromagnetic radiation source.

Both arrangements (b.2) and (b.3) are based on a dynamic chemistry reaction that takes place between a modified nucleobases and a duplex of PNA/target nucleic acid at the gap between that acts as "chemical pocket". The modified nucleobase base contains a chemical group that can react with the PNA probe to form a covalent bond. A reversible reaction then occurs between the formed duplex and the missing nucleobase in the PNA in a thermodynamic process controlled by the selection of the nucleic acid opposite the "chemical pocket", which avoids the incorporation of an incorrect base. This process means that the PNA-target nucleic acid hybridization complex acts as a "template" driving the selection of the fully complementary nucleobase. When the aldehyde group of the nucleobase reacts with the secondary amine of the target, an imine is formed, but since this is a reversible process, it is necessary to use a reducing agent, such as sodium cyanoborohydride, which reduces the formed imine to an amine, preventing the reaction from being reversed and the nucleobase from leaving the "empty" position.

In a particular embodiment of the invention the capture element is selected from an antibody, a receptor, a peptide, a protein, a carbohydrate, or a modified nucleobase. In the preferred embodiment of the invention the capture element is an antibody when used in the arrangements (b.1) and (b.2) or a modified nucleobase, in particular a biotinylated nucleobase, when used in arrangement (b.3).

In a further particular and preferred embodiment of the invention the detection elements are an antibody or a PNA, which in each case have bound thereto a nanoparticle that is the tag that will be detected after irradiation by the plasmonic effect produced. The detection element is an antibody in arrangement (b.1) whereas the detection element is a PNA in both arrangement (b.2) and (b.3).

In a particular embodiment of the invention, the system of the invention is used to detect at least two target analytes, one of peptidic nature and one of nucleic acid nature. Under this embodiment there are two possible approaches for the system of the invention, namely approach A and B.

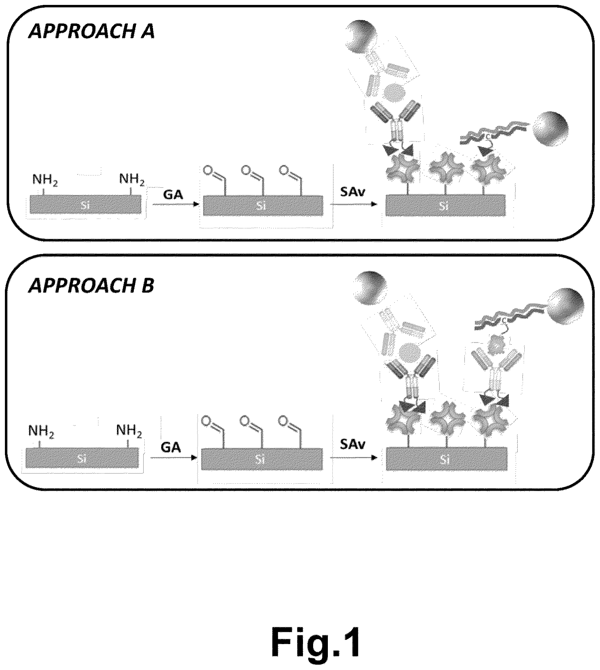

In embodiment or approach A, the system of the invention comprises at least the arrangement of elements (b.1) and (b.3), so that at least a target analyte of peptidic or proteic nature and a target analyte of nucleic acid is detected. This embodiment is represented in Figure 1 as approach A.

In approach B, the system of the invention comprises the arrangement of at least elements (b.1) and (b.2), so that at least a target analyte of peptidic or proteic nature and a target analyte of nucleic acid is detected. This embodiment is represented in Figure 1 as approach B.

In a particular and preferred embodiment, the system is arranged to detect one target analyte of proteic or peptidic nature and one target analyte of nucleic acid nature. Within this particular embodiment, the system preferably comprises the combination of the arrangement of elements (b.1) and (b.2) (approach B) or alternatively the arrangement of elements (b.1) and (b.3) (approach A).

To the extent that the target analytes are of peptidic or proteic nature or of nucleic acid nature, these can be detected by means of the system of the invention. Without wishing to be a limitative list, the system of the invention may be arranged to detect a target analyte of peptidic nature comprising a viral or bacterial protein, toxins, cancer biomarkers, neurological biomarkers or any other disease biomarker, peptidic hormones or cytokines and target analytes of nucleic acid nature comprising genes, SNPs, ESTs, mRNA, tARN, iRNA, rRNA, miRNAs or mitochondrial DNA.

One of the advantages of the present invention is that the same sample can be used to simultaneously detect the at least one target analyte of peptidic nature and the at least one target analyte of nucleic acid sample. This avoids complex processing and conditioning of the samples for target detection. This makes the handling simpler and reduces the time for detection, which means that the present system is very suitable for implementation in POC devices. Although samples used may be of any kind such as solid samples (i.e. biopsies), in a particular and preferred embodiment the samples are fluid samples. Without being a limitative list, the sample may comprise blood, serum, plasma, urine, saliva, bile, cerebrospinal fluid, semen, or vaginal discharge.

In the system of the invention if the target analytes are present in the sample, even at ultra-low concentrations, they can be detected and quantified based on the scattering intensity produced by the nanoparticles. If the target analyte is not present in the sample, there would be no detectable plasmonic effect on the substrate since nanoparticles will not be present.

The detection and quantification can be performed by measuring the scattering intensity produced by the nanoparticles when the system is irradiated with electromagnetic radiation. The detectable plasmonic effect due to irradiation at any wavelength of the white light spectrum will be different for every type of nanoparticle and so a specific tag for any type of target analyte from the sample.

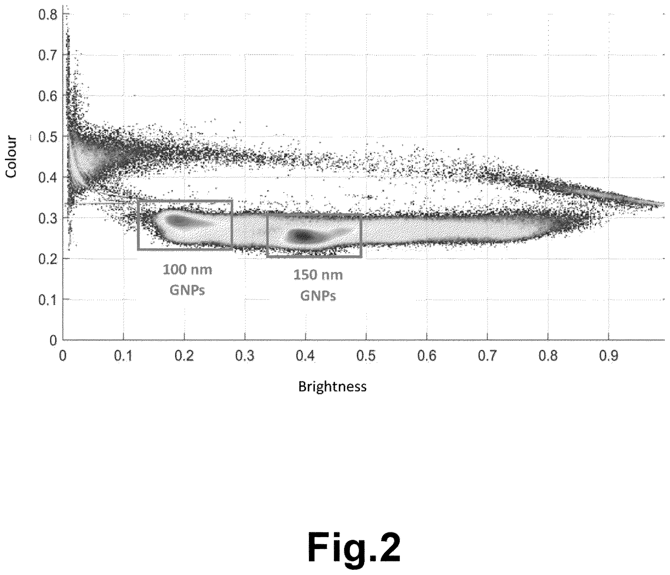

In the system of the invention, nanoparticles can be observed using optical techniques, such as dark-field microspectrophotometry. These observations produce detailed images that clearly showcase bright spots, associated with the presence of nanoparticles on the surface (see figure 2). Currently, it remains challenging to directly ascertain if a specific nanoparticle observed is conjugated with a target analyte. To quantify the number of nanoparticles lacking the target analyte, a comparison is made with a negative control. This control essentially involves an assay where nanoparticles, devoid of any target analyte, are introduced. By using this reference, the level of assay non-specificity can be accurately determined.

The image-processing of the obtained images is fundamental in the system of the invention. It plays a crucial role in digital counting of plasmonic nanoparticles, in distinguishing plasmonic nanoparticles from other residues on the surface that might produce scattering. Additionally, image-processing is essential for enabling multiplexing and for precisely identifying various agglomeration states of the plasmonic nanoparticles. These functionalities underline the indispensable nature of image-processing within this technology, ensuring accurate detection, differentiation, and quantification of target analytes. Detailed methodologies and the importance of image-processing in this context have been well-described in previous patents, such as EP3719477 and EP3719461.

### Method for multiplexed detection and/or quantification

Another aspect of the invention is a method for a multiplexed and simultaneous detection and/or quantification in a sample of a target analyte of peptidic nature and a target analyte of nucleic acid nature based on the biodetection system of the invention which comprises:
a) Simultaneously contacting a sample:
   a.1) with a PNA molecule, as detection element, having bound thereto a nanoparticle with plasmonic properties, the PNA molecule being fully complementary to the target analyte of nucleic acid nature except for a single nucleobase; and
   a.2) with an antibody, as detection element, having bound thereto a nanoparticle which is capable of specifically binding to the target analyte of peptidic nature, wherein the nanoparticle has different plasmonic properties to the one of element a.1);
b) Washing by centrifugation the reaction product resulting from step a) to isolate the elements with nanoparticles,
c) Reacting the product resulting from step b) with either:
   (c.1) a fluorophore-modified nucleobase in the presence of a reducing agent and a buffer at pH 5-7, preferably 6, in case arrangement (b.2) of claim 1, or
   (c.2) a biotinylated nucleobase in the presence of a reducing agent and a buffer at pH 5-7, preferably 6, in case arrangement (b.3) of claim 1;
d) Incubating the reaction product of step c) with a biotinylated antibody as capture element capable of specifically binding to the target analyte of peptidic nature; and also with a biotinylated antibody as capture element capable of specifically binding to the fluorophore in case step (c.1) is followed;
e) Incubating the product resulting from d) in a substrate of dielectric material having a surface functionalized with streptavidin so as to anchor all biotinylated elements, whereby only those detection elements that have specifically bound to their corresponding target analyte will be anchored to the substrate;
f) irradiating the substrate resulting from step e) with an electromagnetic radiation wherein the presence of each target analyte in the sample is detected by the plasmonic effect produced by the nanoparticles which can be measured by optical means, said plasmonic effect being different for each type of target analyte,
g) digitally count plasmonic nanoparticles so that the presence or absence of each target analyte in the sample is identified and quantified by correlation with a calibration curve and comparison with a negative control,
h) optionally acquiring and storing images for further image analysis.

This method will be referred along these lines as the method of the invention or just as the method.

Step a) of the method is the detection step where the target analytes of peptidic and nucleic acid nature, if present in the sample, will bind or hybridize with the detection elements that are present in a reaction medium. The sample can be directly applied in the reaction medium without any kind of pre-processing step. The detection step can be carried out at room temperature although temperature around 30-40°C will favor the antigen capture and the PNA/nucleic acid hybridization. If both target analytes are present in the sample, the resulting product of step a) will be a solution containing an antibody firmly bound to the target analyte of peptidic or proteic nature and a PNA/nucleic acid duplex hybridized except for a one single nucleotide. In addition, both elements of the reaction will also have attached thereto a nanoparticle with different plasmonic properties that was bound to the antibody and the PNA.

Step b) is the washing step and it allows to separate and isolate the elements having nanoparticles by centrifugation. These elements can be either detection elements that have not reacted with their corresponding target analytes or those detection elements that have actually reacted with their corresponding target molecule. The phase containing the nanoparticles is recuperated and resuspended in buffer at a pH 5-7 preferably 6 for further processing in step c).

Step c) is an essential step for the final detection of the target analytes of nucleic acid nature. For the final detection of this type of target analytes, a reaction between the PNA/nucleic acid duplex with a modified nucleobase must take place so that the duplex gets irreversibly linked and functionalized by means of the modified nucleobase. Although the type of reaction will be always the same, the modified nucleobase will be different depending on whether arrangement (b.2) or (b.3) is used for final detection. In this sense, if the system is designed for detecting through an arrangement (b.2), the nucleobase used is a fluorophore modified nucleobase which will need to be further processed in step e) in order to make it able to anchor to the streptavidin functionalized surface of the dielectric material. On the contrary, if the system is designed for an arrangement (b.3) detection, a biotinylated nucleobase is used so that after the reducing reaction takes place a biotinylated PNA/nucleic acid target duplex is obtained. Such a biotinylated product has the capacity to directly bind to the surface. Irrespective of the arrangement used, the duplex PNA/target analyte must be reacted with the modified nucleobase in the presence of a reducing agent such as sodium cyanoborohydride and a buffer that maintains the pH between 7-5 preferably at 6. The solution resulting from step c) contains the antibody bound to the target analyte of peptidic nature and having bound thereto a plasmonic nanoparticle and PNA/nucleic acid target duplex having bound thereto a nanoparticle and being modified with either a fluorophore or a biotin molecule depending on the arrangement used in the system. This solution is further processed in step d).

Step d) has the aim of introducing a biotinylated capture element on those arrangements of elements that still don't have a biotin molecule, namely arrangement (b.1) and (b.2). This will introduce the element that will enable to get anchored to the streptavidin functionalized surface in the next step of the method. In order to carry out this step the solution resulting from step c) is incubated with a biotinylated antibody capable of specifically binding to the target analyte of nucleic acid nature and, in the case arrangement (b.2) is followed for detection, also incubating with a biotinylated antibody capable of specifically binding to the fluorophore. The incubation can be carried out at room temperature although temperature around 30-40°C will favor the antigen capture by the antibodies. In the case where the target analytes either of peptidic or nucleic acid are present in the sample, the solution resulting from step c) contains biotinylated elements irrespective of the arrangement used.

Step e) comprises incubating the biotinylated reaction products resulting from all the previous steps in the substrate of dielectric material functionalized with streptavidin. Where the sample contains the target analyte, this will result in the anchorage of the different arrangement of elements in the surface of the substrate of dielectric material. Depending on the concentration of the target analyte the anchorage of elements in the surface will be higher or lower and so they will give a different picture once detection is carried out by irradiation with an electromagnetic source.

Step f) involves irradiation with an electromagnetic source. This irradiation induces a distinct plasmonic response in each nanoparticle, allowing them to be differentiated from others based on their unique optical signatures. This characteristic plasmonic effect essentially acts as a 'tag' for each target analyte. After light irradiation, the presence of each nanoparticle associated to each different type of analyte can be visualized using optical methods, such as dark-field microspectrophotometry. Image-processing is mandatory in order to perform identification, localization, characterization, classification and digital counting of plasmonic nanoparticles, , distinguishing plasmonic nanoparticles from other residues, identifying various agglomeration states, and enabling effective multiplexing.

Step g) implies digitally counting of plasmonic nanoparticles in order to detect the presence and the abundance or concentration of the target analyte in the sample. In this step, image analysis becomes instrumental in identifying nanoparticles linked to various types of analytes within the sample. While the immediate detection is centered on the nanoparticles, it's necessary to correlate this data to the actual concentration of the target analytes. This correlation is carried out by juxtaposing the observed signal intensity with a calibration curve. Derived from the assay, this calibration curve correlates the count of nanoparticles with predetermined analyte concentrations, facilitating precise quantification of the analyte in the sample. Additionally, the negative control within the calibration curve plays a crucial role in discerning the quantity of nanoparticles that adhere non-specifically to the surface, namely those nanoparticles not bound to any target analyte.

Image analysis can be carried out by any suitable means capable of processing the image so as to produce a result. In the context of the invention, it is especially preferred to use a technology analogous to that disclosed in EP3719477 for detection and quantification of target analytes.

In a particular embodiment of the method, the image analysis is a software analysis of the spatially and spectrally resolved images taken with the optical means and comprises:
a) reading data of the acquired images from storage means;
b) correcting the read data to reduce inhomogeneities and noise of the acquired images;
c) localizing particles in the acquired images using the corrected data to obtain a position for each particle;
d) characterizing each particle individually to obtain an intermediate analysis result which comprises the position and characterization parameters for each particle;
e) classifying the particles based on the characterization parameters of each particle to obtain classes of particles;
f) counting a number of particles per class for each acquired image;
g) calculating an overall analysis result which comprises at least one statistical value, which is calculated for each biomarker in each sample of the biosensor using the number of particles per class for all the images acquired from the same sample, wherein the at least one statistical value per sample is correlated with an indication of the presence of a biomarker in the sample.

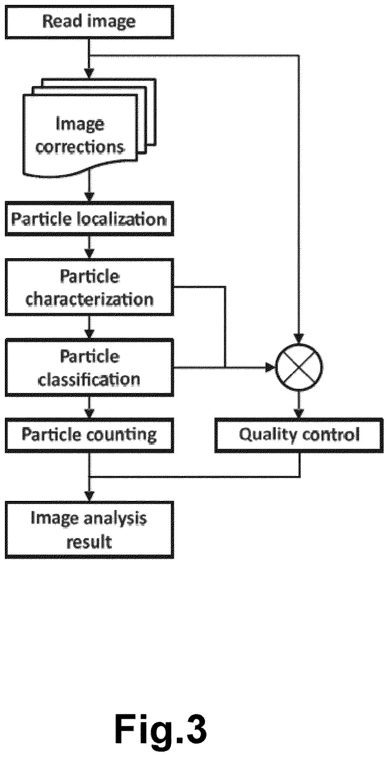

The flowchart summarizing the different steps for the image analysis is represented in figure 3.

In a particular embodiment, the method of the invention may contain two optional washing steps after steps c) and d) of the method. These washing steps are analogous to step b) of the method and require centrifugation of the reaction product resulting from steps c) and/or d) in order to isolate the arrangements containing the nanoparticles. As in step b) the phase containing the nanoparticles is recuperated and resuspended in buffer at a pH 5-7 preferably 6 for further processing the subsequent steps.

In a particular embodiment the dielectric material used in the method of the invention is selected from silicon oxide, silicon, polymers or hydrogels.

The plasmonic resonance peak is highly dependent on the nanoparticle size and so the nanoparticles used in the method must have at least one of its dimensions with a size of 2 nm to 300 nm, preferably 5 nm to 200 nm.

Regarding the material of the plasmonic nanoparticles, in a particular embodiment these are gold nanoparticles, silver nanoparticles or nanoparticles of plasmonic metamaterial such as, but not limited to, titanium nitride and non-stoichiometric oxides such as non-stoichiometric vanadium, titanium and aluminum oxides. A preferred embodiment to be used in the method of the invention is represented by gold nanoparticles.

Furthermore, the nanoparticle can adopt, in the method of the invention, a plurality of forms or structures. In a particular embodiment, the nanoparticles are nanospheres, nanorods, pointed nanorods, nanoshells, nanocages/frames, hollow nanospheres, tetrahedra, octahedra, cubes, icosahedra, rhombic dodecahedra, concave nanocubes, tetrahexahedra, obtuse triangular bipyramids, trisohectahedra and nanoprisms.

### Biosensing platform

An additional aspect of the invention is a biosensing platform for simultaneous, multiplexed, high throughput and ultra-sensitive optical detection of biomarkers labelled with plasmonic nanoparticles comprising the biodetection system of the invention as biosensor (BS).

The biosensing platform of the invention in addition to the biosensor based in the biodetection system of the invention further comprises a broadband and continuous spectrum illumination system (IS), an optical detector (OD) for simultaneously capturing and resolving spatially and spectrally the scattering signal of each individual nanoparticle, an autofocus system (AF) and an optical system (OS) adapted to collect the scattered light from the biosensor's surface onto the optical detector (OD), the platform being provided with translation means (MS) for the movement of the optical system and/or the biosensor, such that the optical system (OS) and the biosensor (BS) can be displaced relative to each other in the three dimensions, and wherein the processing means are adapted to:
i) simultaneously capture spatially and spectrally resolved scattering signals from each nanoparticle individually, and
ii) to analyze these signals simultaneously with the capture process.

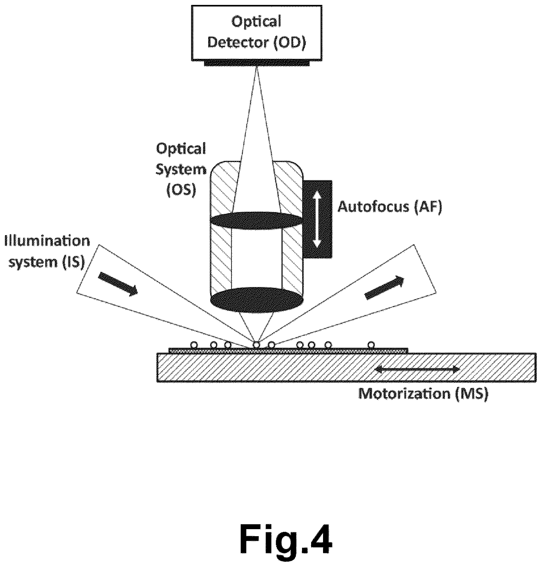

The biosensing platform of the invention is schematically represented in figure 4.

The illumination system (IS) is composed of, for example, a broadband VI-NIR light source coupled to an illuminator setup. The term "broad" means that the light source presents a very broad spectral emission in the visible (400 nm - 700 nm) and/or in the near infrared spectral range (from 700 nm to 1000 nm). For the purpose of the invention, a broadband light source could be a tungsten halogen lamp, a Xenon lamp, a super-continuum laser or a combination of multiple LEDs or LASERs. The illuminator setup collects the light coming from the broadband light source and illuminates the sample surface at glazing angle with a well collimated light spot.

The main purpose of the optical system is to recover in a very efficient way the weak scattering light coming from the sample surface. The optical system (OS) in a preferential design of the invention could be an infinity-corrected optical system that is basically obtained by combining an optical objective with a tube lens. Although not optimal, the optical system (OS) could be designed with just an optical objective (finite-corrected optical system).

The optical detector (OD) captures the scattered light collected by the optical system and it has the capability to simultaneously resolve the optical signals coming from the sample surface with high spatial and spectral resolution. In order to perform spatial and spectral analysis of a sample simultaneously, it is necessary that the optical detector is a micrometrically dense array of spectrometers. The dense array gives the capability to analyze the different spatial positions of the sample simultaneously, while each spectrometer of the array allows a spectral analysis in each point of the sample. The optical detector described here allows the simultaneous analysis of a sample both in the spatial and in the spectral coordinates. The dense array of spectrometers could be realized i) with an array of photodetectors coupled with an array of optical filters arranged in a mosaic pattern, ii) with multiple arrays of photodetectors coupled with dichroic optical filters or iii) with a vertically stacked array of photodetectors able to detect different spectral bands at the same spatial position. These types of optical detectors are a viable technological solution for the simultaneous spatial and spectral analysis of a sample with sub-micrometrical resolution and at multiple spectral bands (typically at least 2 and not more than 30). Preferentially, an optical detector with a high dynamic range should be used, allowing to image nanoparticles associated with different biomarkers with only one capture, such that the time required for the measurement process does not increase in case of using more than one biomarker.

To focus the sample surface in a fast and automatic way, the optical system is equipped with an autofocus system (AF); the automatic focusing of the sample can be achieved either via hardware or via software schemes. Hardware-based autofocusing systems, commonly known in literature as active autofocusing systems, are commonly obtained by projecting laser light onto the sample (the laser being part of the AF) and collecting the laser light reflected from the sample with a differential photodetector. By measuring the position of the reflected light on the photodetector, it is possible to quantify the distance of the sample surface from the best focusing position; the optimum position is therefore recovered with one or more motorized stages able to modify the relative distance between the sample and the head of the optical scanner by moving the biosensor, the optical system or part of it, or both hardware components. To precisely focus the sample surface, the typical resolution of the movement needs to be far below the depth of field of a high-resolution optical objective (which typically could reach down to 200 nm for a 100X optical objective). Many different technological solutions can be implemented, such as stepper or DC motors, or piezoelectric actuators.

Software-based autofocusing systems capture several images of the sample at different relative distances between the sample and the optical head; the capture of images can be performed with the same optical detector that is used for the optical analysis of the sample, or with a dedicated one. The different captured images are analyzed with dedicated algorithms that calculate the contrast of each image and can quantify the distance of the sample surface from the best focusing position. The best position is finally recovered with one or more motorized stages able to change the relative distance between the sample and the optical head of the scanner.

As measurements need to be performed on very extended surface areas (hundreds of cm²), the platform also requires the use of a motorization system (MS) able to modify the relative position between the biosensor surface and the optical system.

This task can be achieved with many different experimental configurations:
A. The sample is moved along two axes; the optical system remains stationary.
B. The optical system is moved along two axes; the sample holder remains stationary.
C. Both the optical system and the sample are moved, e.g., the optical system along one axis, and the sample holder along a second axis.

### Analysis software

In addition to the hardware components and the software to control these components, the platform requires software to analyze the images taken with the optical detector (OD), in the following referred to as analysis software (analysis SW).

Preferably, the analysis SW runs in parallel to the image acquisition of the platform, so that the results from the image analysis are obtained shortly after the end of the acquisition (the "scan"), thereby minimizing the time-to-results. Alternatively, the analysis SW can be executed once the acquisition has been finished or starting at any point during the acquisition.

To reduce the time needed for the analysis, multiple images can be analyzed in parallel. A highly efficient image analysis is implemented, which allows the images to be handled independently for most of the analysis.

For the image analysis, each image is read and, if needed, corrections are applied (correction of background and of inhomogeneities etc., e.g., a dark-current correction, or a flat-field correction). The core of the analysis consists in the recognition, the classification, and the counting of particles. To do this, the particles are first localized in the image sample, they are characterized (brightness, emission spectrum etc.), the results are used to classify the particles (nano-particle monomer, cluster, dust etc.), and finally they are counted per class. These numbers constitute the principal result for each image; in addition, further results are derived which permit a quality control of the measurement (correct focusing, etc.). Since each type of plasmonic nanoparticle is associated specifically with a different biomarker, from the numbers of the different particles in each image sample, the concentrations of the respective biomarkers in the corresponding sample can be deduced, e.g., the number of nanoparticles of one type related to the capturing of a peptidic biomarker, and the number of a second nanoparticle type related to the capturing of a nucleic acid biomarker.

The image analysis comprises the following steps:
a) reading data of the acquired images from storage means;
b) preprocessing of the read data, potentially comprising any of the following options, in a suitable order:
   - correcting the read data to reduce inhomogeneities (e.g., in brightness and/or color) and noise of the acquired images;
   - binning of the images to reduce their size;
   - modification of the images' gamma value (e.g., to obtain images which are linear in brightness);
   - modification of the images' color space, e.g., transformation from RGB to HSV, L*a*b etc., or to other representations, for example, normalized brightnesses (e.g., on a scale of zero to one, with 0 = black and 1 = white) and relative colors (e.g., relative red = R / (R+G+B));
c) localizing particles in the acquired images using the corrected data to obtain a position for each particle, where localization is obtained, for example, in the following way:
   - creation of a pattern (or several patterns) which represents the shape and size of a nanoparticle (or other particle),
   - calculation of the two-dimensional correlation between the pattern and each of the acquired images, such that a high correlation value indicates that the image at this position contains a structure which is probably a particle,
   - localization of the local maxima of the correlation, and using the position of these maxima as particle positions,
d) characterizing each particle individually to obtain an intermediate analysis result which comprises the position and characterization parameters for each particle, where the characterization parameters comprise any of the following:
   - brightness, for example in case of a HSV color space the "V" value averaged over the extend of the particle,
   - color, for example in case of a HSV color space the "H" (=hue) value averaged over the extend of the particle,
   - size, for example the full width at half maximum (FWHM) of the particle,
   - shape, for example values which indicate if the particle appears like a peak in the image (maximum brightness at the center) or like a donut (where the brightness exhibits a local minimum at the center),
e) classifying the particles based on the characterization parameters of each particle to obtain classes of particles;
f) counting a number of particles per particle class for each acquired image;
g) calculating an overall analysis result which comprises at least one statistical value, which is calculated, for each biomarker in each sample of the biosensor, by using the number of particles per particle class for all the images acquired from the same sample, and the statistical value calculated per sample being correlated with an indication of the presence or the concentration or both of a biomarker in the sample.

The classification applied by the analysis SW of the platform typically uses the two particle characteristics "brightness" and "color" to classify the particles, resulting in the following classes:
(1) noise (very low brightness),
(2) residues from the biosensor fabrication (certain "color"),
(3) dust (different "color"),
(4) individual nanoparticles (distinct "color" and brightness),
(5) clusters of nanoparticles (dimers, trimers etc., based on "color", brightness). For these clusters, their brightness typically depends systematically on the brightness of the corresponding individual nanoparticle (monomer). This information is used to improve the classification.

Using the two characteristics "brightness" and "color" (or other pairs of characteristics, or more than two), the classification is basically a segmentation of the parameter space, for example, all particles with a brightness and a color in a certain range (e.g., brightness from 0.1 to 0.2 and color from 0.35 to 0.45, both on a scale from zero to one) are classified as "monomers of nanoparticles". While the segmentation in this example uses the parameters brightness and color independently, thereby defining a "rectangle" in the axes color vs. brightness, they can also be dependent, resulting in an area with borders which are not parallel to the axes.

The particle classification is of particular importance in the case of a multiplex detection like the one considered in this invention (detect one target analyte of peptidic nature and at least one target analyte of nucleic acid nature from the same sample). In this case, two or more different nanoparticles are used (one nanoparticle type for each target molecule type). All need to be distinguished from particles which are not nanoparticles, and at the same time the different types of nanoparticles need to be differentiated one from the other.

Therefore, additional aspects are considered by the analysis SW:
- if only monomers of nanoparticles are of interest, and monomers show a donut shape as a distinguishing feature, this parameter can be used to eliminate all non-donut shaped particles;
- in the case that certain classes overlap significantly in the parameter space, e.g., the clusters (dimers, trimers etc.) of a less-bright nanoparticle with the monomers of a different (brighter) nanoparticle, the calculated number of particles can be corrected considering the following:
   a) Measurements in which only one type of nanoparticle is present are used to calculate the ratios between monomers, dimers, trimers etc. of each type of nanoparticle used in the multiplex.
   b) For these particles, it is calculated which fraction of them would fall into the regions of the parameter space which belong to the further types of nanoparticles used.
   c) Starting, e.g., with the monomers of lowest brightness, their number is used to estimate the numbers of corresponding dimers, trimers etc., and how many of them appear in the regions of the parameter space corresponding to the other nanoparticles.
   d) These numbers are used to correct the number of particles counted in a certain region, i.e., the number of clusters is subtracted from the uncorrected count.
   e) The procedure is repeated for the rest of the nanoparticles, e.g., in order of increasing brightness.
- In case of substantial overlap between the distributions from two (or more) types of particles in the parameter space, the assignment of a simple line between them might result in a significant number of wrongly classified particles. This can be reduced with an alternative approach: For each particle type of interest, a function is defined which matches the shape of the distribution in the parameter space (like a "peak" in a spectrum), where the spectrum can have one (e.g., brightness) or more dimensions (e.g., brightness and color). To each of these functions (one for each particle type) a weight is assigned, and, in an optimization procedure, the weights are adjusted such that the weighted sum of the functions best matches the distribution obtained for a certain sample (either for each image, or from all images belonging to the same sample etc.). From these optimum weights the number of each of the particle types can be calculated, as an alternative to the previously explained counting procedure.

### EXAMPLES

### Example 1: Proof of concept 1 (approach A)

### 1.1-Use of miR-122 and IL6 and their importance in DILI

Approach A of the simultaneous plasmonic detection platform using dynamic chemistry (figure 1) has been tested using miR-122 and interleukin 6 (IL-6). miR-122 has been established as a valuable indicator for evaluating drug-induced liver injury (DILI), being released into de bloodstream by liver cells [28, 29]. Furthermore, IL-6 is involved in the liver's inflammatory response to drug-induced damage, with elevated IL-6 levels associated with liver inflammation and DILI progression [29, 30]. Simultaneous detection of these biomarkers of distinct nature allows for a precise and comprehensive assessment of liver status and the severity of hepatic damage. This innovative approach not only enhances the accuracy of early DILI diagnosis but also has the potential to influence treatment decisions. The capability to simultaneously identify and quantify miR-122 and IL-6 provides valuable clinical insights, enabling swifter and more effective medical intervention for DILI patients.

### 1.2-Manufacturing of GNPs

100 nm gold nanoparticles (GNPs) at 192.3 µg/ml were incubated in MES pH = 3.8 with PNA - 122 (SEQ ID NO 2) at 270 nM, and with 1 mg/ml EDC and 1.5 mg/ml sulfo-NHS in a 1:1 ratio. PNA-122 is a shorter PNA sequence complementary to miR - 122 (SEQ ID NO 1), differing in its base sequence in one single nucleotide from the sequence of miR-122, because it lacks a base (abasic position).These PNA - 122 coated gold nanoparticles were blocked with 1 mg/ml methyl - PEG - SH in 50 mM Tris - Tween20 0.1% and stored in 2 mg/ml methyl - PEG - SH in 50 mM Tris - Tween20 0.1%.

150 nm gold nanoparticles at 228.35 µg/ml were incubated in MES pH = 3.8 with anti - IL - 6 detection antibody at 3.85 µg/ml and with BSA at 73.08 µg/ml, and 1 mg/l EDC and 1.5 mg/ml sulfo-NHS in a 1:1 ratio. These anti - IL-6 coated gold nanoparticles were blocked with 1 mg/ml BSA in PBS - Tween 0.05% and stored in 2 mg/ml BSA in PBS - Tween20 0.05%.

All incubations in GNPs manufacturing were performed for 1 hour at room temperature with agitation. Between incubations, the gold nanoparticles were washed by centrifugation for 5 minutes at 4°C and 13500 rpm, 3 times. After the first incubation with the antibody or PNA, the washes were made with the blocking solution, and after the second incubation with the blocking solution, the washes were made with the storage solution, with which they were finally stored at 4°C until use.

### 1.3- Manufacturing of silicon surfaces

Silicon surfaces with oxide were silanized by chemical vapor deposition with (3-aminopropyl) triethoxysilane (APTES). Then, first they were incubated with 5% glutaraldehyde and second with 3 µg/ml streptavidin, both in PBS. Finally, the surfaces were blocked with 10 mg/ml BSA in PBS. All these incubations were done for 1 hour at 37°C, and between the incubations, 5-minute washes with PBS were done: 4 after glutaraldehyde, 3 after streptavidin and 2 after blocking. Finally, 2 washes with miliQ water were done, and the surfaces were dried with a nitrogen gun to store them at 4°C until use.

### 1.4- Combined detection assay

In a first step, 150 GNPs with anti - IL - 6 antibody (9 million - 18%) and 100 nm GNPs with PNA - 122 (41 million - 82%) were incubated for 1 hour at 800 rpm and 37°C for IL - 6 capture and miR-122 hybridization, respectively. There were two conditions, one negative without IL- 6 and without miR - 122, and another positive with IL-6 at 150 pg/ml and with miR - 122 at 300 pM. Then, they were washed by centrifugation for 5 minutes at 13500 rpm and 4°C, just one time. These GNPs were resuspended in DR buffer with pH = 6 (provided by Destina Genomic) with 5 µM biotinylated nucleobase and 1 mM reducing agent for 1 hour at 800 rpm and 40°C (Destina Reaction). Then, they were washed by centrifugation for 5 minutes at 13500 rpm and 4°C, 2 times.

In the third step, the GNPs were incubated with the anti - IL - 6 capture antibody at 225 ng/ml in 1% BSA in PBS -Tween20 0.05% for 1 hour at 800 rpm and 37°C. The GNPs were then washed by centrifugation for 5 minutes at 13500 rpm and 4°C, just one time. Finally, the GNPs were resuspended in 1% BSA in PBS - Tween20 0.05% and incubated on the silicon surface with streptavidin for 1 hour at 37°C (100 µl/well).

### 1.5- Test measurement and analysis

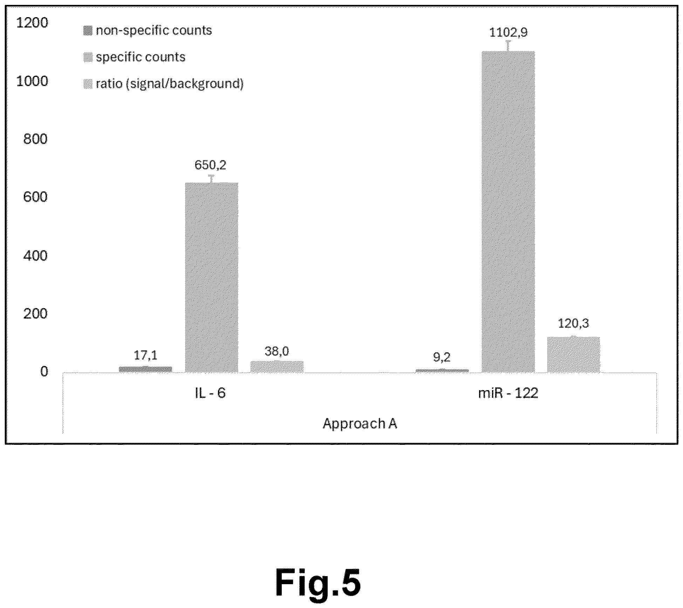

The test was measured by image analysis (AVAC analyzer from Mecwins), obtaining a number of nanoparticles per well (average of all photos taken of the well) of each size (100 nm for miR - 122 and 150 nm for IL - 6) for the positive condition and for the negative condition. In this way, for each biomarker, 3 results were obtained: non - specific counts (background), specific counts (signal) and ratio (signal / background). With this approach, the IL - 6 and miR - 122 simultaneous detection works because with both biomarkers a ratio (signal / background) > 2 was obtained. The IL - 6 ratio is lower than the miR - 122 ratio, so this approach it more optimal for the nucleic acids detection than for the proteins detection (figure 5).

### Example 2: Proof of concept 2 (approach B)

### 2.1- Use of miR-122 and IL6 and their importance in DILI

Approach B of the simultaneous plasmonic detection platform using dynamic chemistry (figure 1) has been tested using miR-122 and interleukin 6 (IL-6). miR-122 has been established as a valuable indicator for evaluating drug-induced liver injury (DILI), being released into de bloodstream by liver cells [28, 29]. Furthermore, IL-6 is involved in the liver's inflammatory response to drug-induced damage, with elevated IL-6 levels associated with liver inflammation and DILI progression [29, 30]. Simultaneous detection of these biomarkers of distinct nature allows for a precise and comprehensive assessment of liver status and the severity of hepatic damage. This innovative approach not only enhances the accuracy of early DILI diagnosis but also has the potential to influence treatment decisions. The capability to simultaneously identify and quantify miR-122 and IL-6 provides valuable clinical insights, enabling swifter and more effective medical intervention for DILI patients.

### 2.2-Manufacturing of GNPs

100 nm gold nanoparticles (GNPs) at 192.3 µg/ml were incubated in MES pH = 3.8 with PNA - 122 (SEQ ID NO 2) at 270 nM, and with 1 mg/ml EDC and 1.5 mg/ml sulfo-NHS in a 1:1 ratio. PNA-122 is a shorter PNA sequence complementary to miR - 122 (SEQ ID NO 1), differing in its base sequence in one single nucleotide from the sequence of miR-122 because it lacks a base (abasic position). These PNA- 122 coated gold nanoparticles were blocked with 1 mg/ml methyl - PEG - SH in 50 mM Tris - Tween20 0.1% and stored in 2 mg/ml methyl - PEG - SH in 50 mM Tris - Tween20 0.1%.

150 nm gold nanoparticles at 228.35 µg/ml were incubated in MES pH = 3.8 with anti - IL - 6 detection antibody at 3.85 µg/ml and with BSA at 73.08 µg/ml, and 1 mg/l EDC and 1.5 mg/ml sulfo-NHS in a 1:1 ratio. These anti - IL-6 coated gold nanoparticles were blocked with 1 mg/ml BSA in PBS - Tween 0.05% and stored in 2 mg/ml BSA in PBS - Tween20 0.05%.

All incubations in GNPs manufacturing were performed for 1 hour at room temperature with agitation. Between incubations, the gold nanoparticles were washed by centrifugation for 5 minutes at 4°C and 13500 rpm, 3 times. After the first incubation with the antibody or PNA, the washes were made with the blocking solution, and after the second incubation with the blocking solution, the washes were made with the storage solution, with which they were finally stored at 4°C until use.

### 2.3- Manufacturing of silicon surfaces

Silicon surfaces with oxide were silanized by chemical vapor deposition with (3-aminopropyl) triethoxysilane (APTES). Then, first they were incubated with 5% glutaraldehyde and second with 3 µg/ml streptavidin, both in PBS. Finally, the surfaces were blocked with 10 mg/ml BSA in PBS, and then, they were incubated with anti - FITC and anti - IL - 6 capture antibody, both at 3.5 µg/ml in PBS. All these incubations were done for 1 hour at 37°C, and between the incubations, 5-minute washes with PBS were done: 4 after glutaraldehyde, 3 after streptavidin, 3 after blocking, and 2 after antibodies. Finally, 2 washes with miliQ water were done, and the surfaces were dried with a nitrogen gun to store them at 4°C until use.

### 2.4 Combined detection assay

In a first step, 150 nm GNPs with anti - IL-6 antibody (9 million - 18%) and 100 nm GNPs with PNA - 122 (41 million - 82%) were incubated for 1 hour at 800 rpm and 37°C for IL - 6 capture and hybridization to the miR-122, respectively. There were two conditions, one negative without IL- 6 and without miR - 122, and another positive with IL-6 at 150 pg/ml and with miR - 122 at 300 pM. Then, they were washed by centrifugation at 13500 rpm for 5 minutes at 4°C, just one time. These GNPs were resuspended in DR buffer with pH = 6 (provided by Destina Genomic) with 5 µM FITC-modified nucleobase and 1 mM reducing agent for 1 hour at 800 rpm and 40°C (Destina Reaction). Then, they were washed by centrifugation for 5 minutes at 13500 rpm and 4°C, 2 times.

Finally, the GNPs were resuspended in 1% BSA in PBS - Tween20 0.05% and incubated on the silicon surface with anti - FITC and anti - IL -6 antibodies for 1 hour at 37°C (100 µl/well).

### 2.5- Test measurement and analysis

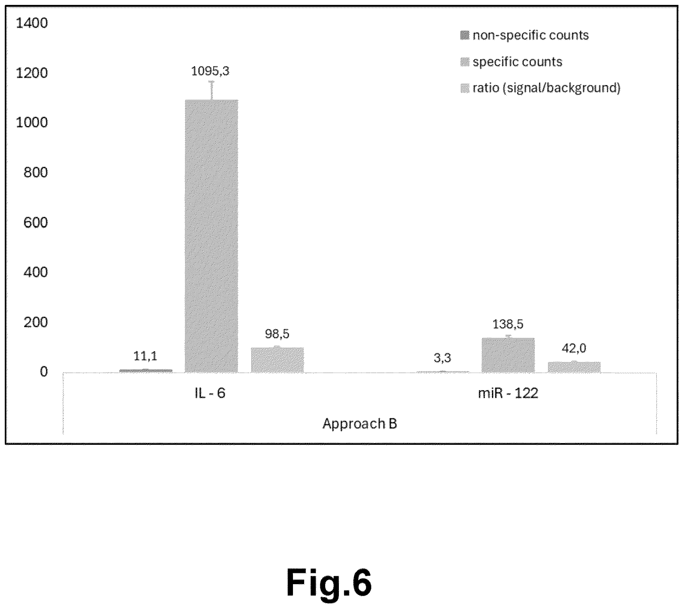

The test was measured by image analysis (AVAC analyzer from Mecwins), obtaining a number of nanoparticles per well (average of all photos taken of the well) of each size (100 nm for miR - 122 and 150 nm for IL - 6) for the positive condition and for the negative condition. In this way, for each biomarker, 3 results were obtained: non - specific counts (background), specific counts (signal) and ratio (signal / background). With this approach, the IL - 6 and miR - 122 simultaneous detection works because with both biomarkers a ratio (signal / background) > 2 was obtained. The miR - 122 ratio is lower than the IL - 6 ratio, so this approach it more optimal for the proteins detection than for the nucleic acids detection (figure 6).

### Example 3: Proof of concept 3 optimization of approach B

### 3.1 Use of miR-122 and IL6 and their importance in DILI

Approach B of the simultaneous plasmonic detection platform using dynamic chemistry (figure 1) has been tested using miR-122 and interleukin 6 (IL-6). miR-122 has been established as a valuable indicator for evaluating drug-induced liver injury (DILI), being released into de bloodstream by liver cells [28, 29]. Furthermore, IL-6 is involved in the liver's inflammatory response to drug-induced damage, with elevated IL-6 levels associated with liver inflammation and DILI progression [29, 30]. Simultaneous detection of these biomarkers of distinct nature allows for a precise and comprehensive assessment of liver status and the severity of hepatic damage. This innovative approach not only enhances the accuracy of early DILI diagnosis but also has the potential to influence treatment decisions. The capability to simultaneously identify and quantify miR-122 and IL-6 provides valuable clinical insights, enabling swifter and more effective medical intervention for DILI patients.

### 3.2- Manufacturing of GNPs

100 nm gold nanoparticles (GNPs) at 192.3 µg/ml were incubated in MES pH = 3.8 with PNA - 122 (SEQ ID NO 2) at 270 nM, and with 1 mg/ml EDC and 1.5 mg/ml sulfo-NHS in a 1:1 ratio. PNA-122 is a shorter PNA sequence complementary to miR - 122 (SEQ ID NO 1), differing in its base sequence in one single nucleotide from the sequence of miR-122 because it lacks a base (abasic position).These PNA - 122 coated gold nanoparticles were blocked with 1 mg/ml methyl - PEG - SH in 50 mM Tris - Tween20 0.1% and stored in 2 mg/ml methyl - PEG - SH in 50 mM Tris - Tween20 0.1%.

150 nm gold nanoparticles at 228.35 µg/ml were incubated in MES pH = 3.8 with anti - IL - 6 detection antibody at 3.85 µg/ml and with BSA at 73.08 µg/ml, and 1 mg/l EDC and 1.5 mg/ml sulfo-NHS in a 1:1 ratio. These anti - IL-6 coated gold nanoparticles were blocked with 1 mg/ml BSA in PBS - Tween 0.05% and stored in 2 mg/ml BSA in PBS - Tween20 0.05%.

All incubations in GNPs manufacturing were performed for 1 hour at room temperature with agitation. Between incubations, the gold nanoparticles were washed by centrifugation for 5 minutes at 4°C and 13500 rpm, 3 times. After the first incubation with the antibody or PNA, the washes were made with the blocking solution, and after the second incubation with the blocking solution, the washes were made with the storage solution, with which they were finally stored at 4°C until use.

### 3.3- Manufacturing of silicon surfaces

Silicon surfaces with oxide were silanized by chemical vapor deposition with (3-aminopropyl) triethoxysilane (APTES). Then, first they were incubated with 5% glutaraldehyde and second with 3 µg/ml streptavidin, both in PBS. Finally, the surfaces were blocked with 10 mg/ml BSA in PBS, and then, they were incubated with anti - FITC and anti - IL-6 capture antibody in PBS. Different proportions of these antibodies have been tested. Due to the IL - 6 detection worked better than the miR - 122 detection with this approach B in proof of concept 2, two conditions have been tested:
- Anti - FITC antibody at 3.5 µg/ml (50%) and anti - IL - 6 capture antibody at 3.5 µg/ml (50%).
- Anti - FITC antibody at 6 µg/ml (86%) and anti - IL - 6 capture antibody at 1 µg/ml (14%).

All these incubations were done for 1 hour at 37°C, and between the incubations, 5-minute washes with PBS were done: 4 after glutaraldehyde, 3 after streptavidin, 3 after blocking, and 2 after antibodies. Finally, 2 washes with miliQ water were done, and the surfaces were dried with a nitrogen gun to store them at 4°C until use.

### 3.4- Combined detection assay

In a first step, 150 nm GNPs with anti - IL-6 antibody (9 million - 18%) and 100 nm GNPs with PNA - 122 (41 million - 82%) were incubated for 1 hour at 800 rpm and 37°C for IL - 6 capture and hybridization to the miR-122, respectively. Different proportions of these GNPs have been tested. Due to the IL - 6 detection worked better than the miR - 122 detection with this approach B in proof of concept 2, two conditions have been tested:
- 41 million of 100 nm GNPs with PNA - 122 (82%) and 9 million of 150 nm GNPs with anti - IL - 6 antibody (18%).
- 45 million of 100 nm GNPs with PNA - 122 (90%) and 5 million of 150 nm GNPs with anti - IL - 6 antibody (10%).

In this test, 7 - point calibration curves were tested, with points with ¼ dilution from 300 pM in the case of miR - 122 and from 150 pg/ml in the case of IL-6, until reaching the negative control.

Then, they were washed by centrifugation for 5 minutes at 13500 rpm and 4°C, just one time. These GNPs were resuspended in DR buffer with pH = 6 (provided by Destina Genomic) with 5 µM FITC-modified nucleobase and 1 mM reducing agent for 1 hour at 800 rpm and 40°C (Destina Reaction). Then, they were washed by centrifugation for 5 minutes at 13500 rpm and 4°C, 2 times.

Finally, the GNPs were resuspended in 1% BSA in PBS - Tween20 0.05% and incubated on the silicon surface with anti - FITC and anti - IL -6 antibodies for 1 hour at 37°C (100 µl/well).

### 3.5- Tested conditions

Therefore, four conditions have been tested in this proof of concept:
- Standard: proportions of GNPs (82% - 18%) and antibodies (50% - 50%) used in the proof of concept 2.
- GNPs - PNA 122: only the proportion of GNPs has changed (90% - 10%).
- Anti - FITC: only the proportion of antibodies has changed (86% - 14%).
- GNPs - PNA 122 + anti - FITC: both proportions of GNPs (90% - 10%) and (86% - 14%) antibodies have changed.

### 3.6- Test measurement and analysis

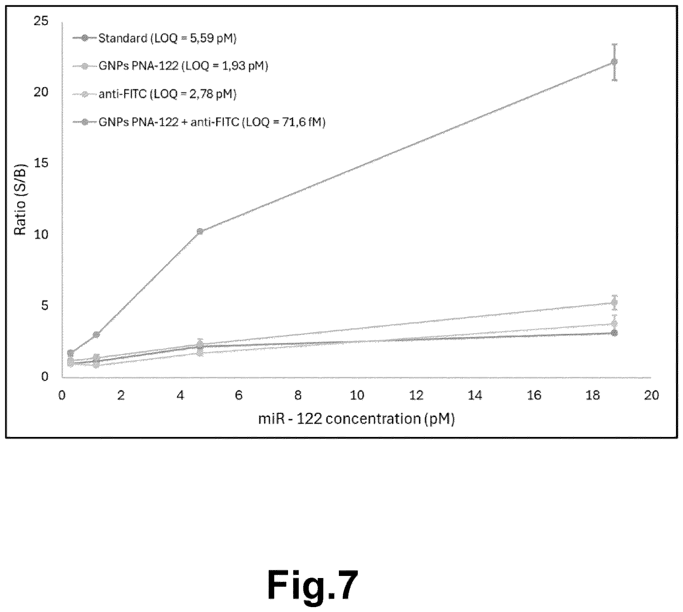

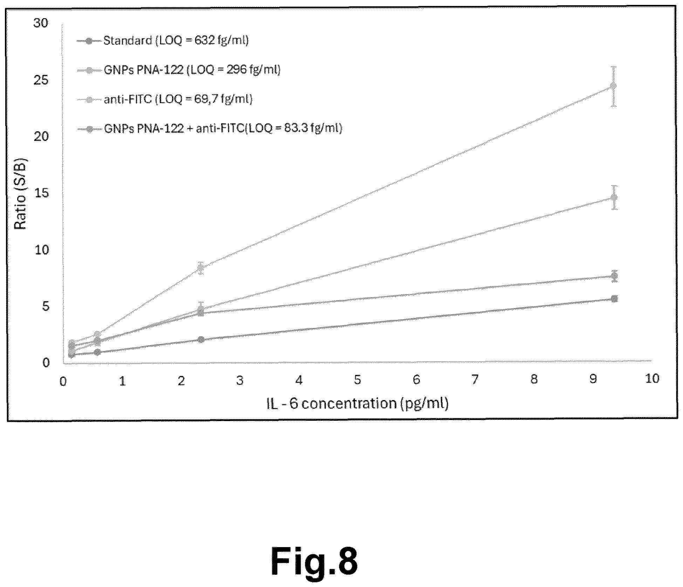

The test was measured by image analysis (AVAC analyzer from Mecwins), obtaining a number of nanoparticles per well (average of all photos taken of the well) of each size (100 nm for miR -122 and 150 nm for IL-6) for all the conditions. In this way, for each biomarker, calibration curves in GNPs number versus the biomarker concentration have been obtained. With this calibration curves, a 5PL adjustment was made, obtaining a limit of quantification (LOQ) value. The best LOQ result with miR - 122 was obtained by increasing both the anti - FITC and GNPs - PNA 122 (figure 7). However, the best LOQ result with IL - 6 was obtained by increasing only the anti - FITC; but the LOQ obtained with IL - 6 by increasing both the anti - FITC and GNPs - PHA is also good (figure 8). So, this condition in which the miR - 122 detection is forced to the maximum by increasing both elements was selected as the best option for the IL - 6 and miR - 122 combined detection with approach B, because the IL - 6 detection continues to function correctly, obtained good sensitivity with both biomarkers. These meticulous optimizations culminated in a remarkable enhancement of sensitivity for approach B of this plasmonic detection platform utilizing dynamic chemistry. Notably, this optimization resulted in a LOQ of 83.3 fg/ml for the IL-6 biomarker and a LOQ of 71.6 fM for nucleic acid (figures 7 and 8).

### References

1. Li J, Peng Y, Liu Y, Li W, Jin Y, Tang Z, Duan Y. Investigation of potential breath biomarkers for the early diagnosis of breast cancer using gas chromatography-mass spectrometry. Clin Chim Acta. 2014 Sep 25; 436:59-67.
2. Saalberg Y, Wolff M. VOC breath biomarkers in lung cancer. Clin Chim Acta. 2016 Aug 1; 459:5-9.
3. Benkoe TM, Mechtler TP, Pones M, Prusa AR, Klebermass-Schrehof K, Rebhandl W, Kasper DC. The plasma activities of lysosomal enzymes in infants with necrotizing enterocolitis: new promising class of biomarkers? Clin Chim Acta. 2015 Jan 1; 438:279-83.
4. Liao Z, Zhang Y, Li Y, Miao Y, Gao S, Lin F, Deng Y, Geng L. Microfluidic chip coupled with optical biosensors for simultaneous detection of multiple analytes: A review. Biosens Bioelectron. 2019 Feb 1; 126:697-706.
5. Gygi SP, Rochon Y, Franza BR, Aebersold R. Correlation between protein and mRNA abundance in yeast. Mol Cell Biol. 1999 Mar; 19(3):1720-30.
6. Flamini E, Mercatali L, Nanni O, Calistri D, Nunziatini R, Zoli W, Rosetti P, Gardini N, Lattuneddu A, Verdecchia GM, Amadori D. Free DNA and carcinoembryonic antigen serum levels: an important combination for diagnosis of colorectal cancer. Clin Cancer Res. 2006 Dec 1; 12(23):6985-8.
7. Khan K, Aslam MA, Zahra FT, Basheer H, Bilal M, Sumrin A. Potential biomarkers for the diagnosis of respiratory tract infection and lungs cancer. Cell Mol Biol (Noisy-le-grand). 2017 Nov 30; 63(11):46-52.
8. Crissman HA, Steinkamp JA. Rapid, simultaneous measurement of DNA, protein, and cell volume in single cells from large mammalian cell populations. J Cell Biol. 1973 Dec; 59(3):766-71.
9. Sansonno D, Cornacchiulo V, Racanelli V, Dammacco F. In situ simultaneous detection of hepatitis C virus RNA and hepatitis C virus-related antigens in hepatocellular carcinoma. Cancer. 1997 Jul 1; 80(1):22-33.
10. Graham ML 2nd, Bunn PA Jr, Jewett PB, Gonzalez-Aller C, Horwitz KB. Simultaneous measurement of progesterone receptors and DNA indices by flow cytometry: characterization of an assay in breast cancer cell lines. CancerRes. 1989 Jul 15; 49(14):3934-42.
11. Ideker T, Thorsson V, Ranish JA, Christmas R, Buhler J, Eng JK, Bumgarner R, Goodlett DR, Aebersold R, Hood L. Integrated genomic and proteomic analyses of a systematically perturbed metabolic network. Science. 2001 May 4; 292(5518):929-34.
12. Mao X, Gurung A, Xu H, Baloda M, He Y, Liu G. Simultaneous detection of nucleic acid and protein using gold nanoparticles and lateral flow device. Anal Sci. 2014; 30(6):637-42.
13. Montserrat Pagès A, Safdar S, Ven K, Lammertyn J, Spasic D. DNA-only bioassay for simultaneous detection of proteins and nucleic acids. Anal Bioanal Chem. 2021 Aug; 413(20):4925-4937.
14. Meena GG, Stambaugh AM, Ganjalizadeh V, Stott MA, Hawkins AR, Schmidt H. Ultrasensitive detection of SARS-CoV-2 RNA and antigen using single-molecule optofluidic chip. APL Photonics. 2021 Jun; 6(6):066101.
15. Zhou J, Wu Z, Hu J, Yang D, Chen X, Wang Q, Liu J, Dou M, Peng W, Wu Y, Wang W, Xie C, Wang M, Song Y, Zeng H, Bai C. High-throughput single-EV liquid biopsy: Rapid, simultaneous, and multiplexed detection of nucleic acids, proteins, and their combinations. Sci Adv. 2020 Nov 20; 6(47):eabc1204.
16. Lin Q, Zhang J, Liu L, Kong J, Fang X. Simultaneous Rapid NucleicAcid and Protein Detection in a Lateral Chromatography Chip for COVID-19 Diagnosis. ACS Omega. 2022 Oct 21; 7(43):38409-38416.
17. Chen F, Zhang F, Liu Y, Cai C. Simply and sensitively simultaneous detection hepatocellular carcinoma markers AFP and miRNA-122 by a label-free resonance light scattering sensor. Talanta. 2018 Aug 15; 186:473-480.
18. Liao Z, Zhang Y, Li Y, Miao Y, Gao S, Lin F, Deng Y, Geng L. Microfluidic chip coupled with optical biosensors for simultaneous detection of multiple analytes: A review. Biosens Bioelectron. 2019 Feb 1; 126:697-706.
19. Dinter F, Burdukiewicz M, Schierack P, Lehmann W, Nestler J, Dame G, Rödiger S. Simultaneous detection and quantification of DNA and protein biomarkers in spectrum of cardiovascular diseases in a microfluidic microbead chip. Anal Bioanal Chem. 2019 Nov; 411(29):7725-7735.
20. Sun W, Song W, Guo X, Wang Z. Ultrasensitive detection of nucleic acids and proteins using quartz crystal microbalance and surface plasmon resonance sensors based on target-triggering multiple signal amplification strategy. Anal Chim Acta. 2017 Jul 25; 978:42-47.
21. Androvic P, Valihrach L, Elling J, Sjoback R, Kubista M. Two-tailed RT-qPCR: a novel method for highly accurate miRNA quantification. Nucleic Acids Res. 2017 Sep 6; 45(15):e144.
22. Chugh P, Dittmer DP. Potential pitfalls in microRNA profiling. Wiley Interdiscip Rev RNA. 2012 Sep-Oct; 3(5):601-16.
23. Godoy PM. et al. Comparison of miRNA Profiling Methods Using Synthetic miRNA Pools and Standardized exRNA Samples Reveals Substantial Performance Differences. bioRxiv. 2019 May 24; pp. 645762.
24. Androvic P, Valihrach L, Elling J, Sjoback R, Kubista M. Two-tailed RT-qPCR: a novel method for highly accurate miRNA quantification. Nucleic Acids Res. 2017 Sep 6; 45(15):e144.
25. López-Gallego F, Guisán JM, Betancor L. Glutaraldehyde-mediated protein immobilization. Methods Mol Biol. 2013;1051:33-41.
26. Li A, Wu Y, Linnoila J, Pulli B, Wang C, Zeller M, Ali M, Lewandrowski GK, Li J, Tricot B, Keliher E, Wojtkiewicz GR, Fulci G, Feng X, Tannous BA, Yao Z, Chen JW Surface biotinylation of cytotoxis T lymphocytes for in vivo tracking of tumor immunotherapy in murine models. Cancer Immunol Immunother. 2016 Dec;65(12):1545-1554.
27. Gabant G, Augier J, Armengaud J. Assessment of solvent residues accessibility using three Sulfo-NHS-biotin reagents in parallel: application to footprint changes of amethyltransferase upon binding its substrate. J Mass Spectrom. 2008 Mar;43(3):360-70.
28. Howell LS, Ireland L, Park BK, Goldring CE. MiR-122 and other microRNAs as potential circulating biomarkers of drug-induced liver injury. Expert Rev Mol Diagn. 2018 Jan; 18 (1):47-54.
29. Sanjay S. Girish C. Role of miRNA and its potential as a novel diagnostic biomarker in drug-induced liver injury. Eur J Clin Pharmacol. 2017 Apr; 73(4):399-407.
30. Zhao L, Wang Y, Zhang Y. The potential diagnostic and therapeutic applications of exosomes in drug-induced liver injury. Toxicol Lett. 2021 Feb 1; 337:68-77.
31. Blennow K, Hampel H, Weiner M, Zetterbeg H. Cerebrospinal fluid and plasma biomarkers in Alzheimer disease. Nat Rev Neurol. 2010 Mar;6(3):131-44.
32. Mielke MM, Hagen CE, Xu J, Chai X, Vemuri P, Lowe VJ, Airey DC, Knopman DS; Roberts RO, Machulda MM, Hack CR Jr, Petersen RC, Dage JL. Plasma phosphortau181 increases with Alzheimer's disease clinical severity and is associated with tau-and amyloid-positron emission tomography. Alzheimers Dement. 2018 Aug;14(8):989-997.
33. Klucherev TO, Olszewski, Shalimova AA, Chubarev VN, Tarasov VV, Attwood MM, Syvänen S, Shiöth HB. Advances in the development of new biomarkers for Alzheimer's disease. Transl Neurodegener. 202 Apr21;11(1):25.
34. Nikolac Perkovic M, Videtic Paska A, Konjevod M, Kouter K, Svob Strac D, Nedic Erjavec G, Pivac N. Epigenetics of Alzheimer's Disease. Biomolecules. 2021 Jan 30;11(2):195.
35. Lee CY, Ryu Is, Ryu JH, Cho HJ. miRNAs as therapeutic tools in Alzheimer's Disease. Int J Mol Sci. 2021 Dec 1;22(23):13012.
36. Duyckaerts C, Delatour B, Potier MC. Classification and basic pathology of Alzheimer disease. Acta Neuropathol. 2009 Jul;118(1):5-36.

## Claims

1. A multiplexed biodetection system arranged to simultaneously detect at least one target analyte of peptidic nature and at least one target analyte of nucleic acid nature from a sample comprising:
a) a substrate of dielectric material having a surface functionalized with streptavidin capable of anchoring biotinylated elements; and
b) at least two of the following arrangement of elements:
(b.1) a biotinylated capture element capable of specifically binding to a target analyte of peptidic nature and a detection element having bound thereto a nanoparticle with plasmonic properties which is capable of specially binding to the target analyte in a sandwich type arrangement;
(b.2) a biotinylated capture element capable of specifically binding to a fluorophore of a fluorophore-modified nucleobase which can only be detected when specific hybridization between a target analyte of nucleic acid nature and a of fully complementary except-for-a-single-nucleobase PNA takes place, wherein the PNA, acting as detection element, has a nanoparticle with plasmonic properties bound thereto and wherein the detection occurs after the fluorophore-modified nucleobase fills the single nucleotide gap between the duplex PNA/target analyte and whereby after a reduction reaction said duplex is irreversibly fixed;
(b.3) a biotinylated nucleobase as capture element which can only be detected when specific hybridization between a target analyte of nucleic acid nature and a of fully complementary except-for-a-single-nucleobase PNA takes place, wherein the PNA, acting as detection element, has a nanoparticle with plasmonic properties bound thereto and wherein the detection occurs after the biotinylated nucleobase fills the single nucleotide gap between the duplex PNA/target analyte and whereby after a reduction reaction said duplex is irreversibly fixed;
with the proviso that the system comprises at least arrangement (b.1) and at least one of arrangements (b.2) or (b.3) and with the proviso that the nanoparticles used in detection of each target analyte have different plasmonic properties.

2. The system according to claim 1, wherein the dielectric material is selected from silicon oxide, silicon, silicon nitride, silicon carbide, graphene, polymers and hydrogels.

3. The system according to any of the previous claims wherein capture element is selected from an antibody, a receptor, a peptide, a protein, a carbohydrate or a modified nucleobase, preferably an antibody or a modified nucleobase.

4. The system according to any of the previous claims wherein the detection element is an antibody or a PNA.

5. The system according to any of the previous claims wherein the plasmonic nanoparticles:
a) have at least one of its dimensions with a size of 2 nm to 300 nm; and/or
b) is a gold nanoparticle, a silver nanoparticle or a nanoparticle of plasmonic metamaterial; and/or
c) has a structure selected from the group of nanospheres, nanorods, pointed nanorods, nanoshells, nanocages/frames, hollow nanospheres, tetrahedra, octahedra, cubes, icosahedra, rhombic dodecahedra, concave nanocubes, tetrahexahedra, obtuse triangular bipyramids, trisohectahedra and nanoprism.

6. The system according to any of the previous claims wherein the target analyte of peptidic nature comprises a viral or bacterial protein, toxins, cancer biomarkers, neurological biomarkers or any other disease biomarker, peptidic hormones or cytokines and target analytes of nucleic acid nature comprises genes, SNPs, ESTs, mRNA, tARN, iRNA, rRNA, miRNAs or mitochondrial DNA.

7. The system according to any of the previous claims where the sample comprises blood, serum, plasma, urine, saliva, bile, cerebrospinal fluid, semen or vaginal discharge.

8. A system according to any of the previous claim comprising:
a) at least the arrangement of elements (b.1) and (b.2); or
b) at least the arrangement of elements (b.1) and (b.3).

9. Method for a multiplexed and simultaneous detection and/or quantification in a sample of a target analyte of peptidic nature and a target analyte of nucleic acid nature based on the system of any of claims 1-8 which comprises:
a) Simultaneously contacting a sample:
a.1) with a PNA molecule, as detection element, having bound thereto a nanoparticle with plasmonic properties, the PNA molecule being fully complementary to the target analyte of nucleic acid nature except for a single nucleobase; and
a.2) with an antibody, as detection element, having bound thereto a nanoparticle which is capable of specifically binding to the target analyte of peptidic nature, wherein the nanoparticle has different plasmonic properties to the one of element a.1);
b) Washing by centrifugation the reaction product resulting from step a) to isolate the elements with nanoparticles,
c) Reacting the product resulting from step b) with either:
(c.1) a fluorophore-modified nucleobase in the presence of a reducing agent and a buffer at pH 5-7, preferably 6, in case arrangement (b.2) of claim 1, or
(c.2) a biotinylated nucleobase in the presence of a reducing agent and a buffer at pH 5-7, preferably 6, in case arrangement (b.3) of claim 1;
d) Incubating the reaction product of step c) with a biotinylated antibody as capture element capable of specifically binding to the target analyte of peptidic nature; and also with a biotinylated antibody as capture element capable of specifically binding to the fluorophore in case step (c.1) is followed;
e) Incubating the product resulting from d) in a substrate of dielectric material having a surface functionalized with streptavidin so as to anchor all biotinylated elements, whereby only those detection elements that have specifically bound to their corresponding target analyte will be anchored to the substrate;
f) irradiating the substrate resulting from step e) with an electromagnetic radiation wherein the presence of each target analyte in the sample is detected by the plasmonic effect produced by the nanoparticles which can be measured by optical means, said plasmonic effect being different for each type of target analyte,
g) digitally count plasmonic nanoparticles so that the presence or absence of each target analyte in the sample is identified and quantified by correlation with a calibration curve and comparison with a negative control and
h) optionally acquiring and storing images for further image analysis.

10. Method according to claim 9, wherein the dielectric material is selected from silicon oxide, silicon, silicon nitride, silicon carbide, polymers, hydrogels or graphene.

11. Method according to any of claims 9-10 wherein each plasmonic nanoparticle with different plasmonic properties has:
a) at least one of its dimensions with a size of 2 nm to 300 nm; and/or
b) is a gold nanoparticle, silver nanoparticle or a nanoparticle of plasmonic metamaterial; and/or
c) a structure selected from the group of nanospheres, nanorods, pointed nanorods, nanoshells, nanocages/frames, hollow nanospheres, tetrahedra, octahedra, cubes, icosahedra, rhombic dodecahedra, concave nanocubes, tetrahexahedra, obtuse triangular bipyramids, trisohectahedra and nanoprisms.

12. Method according to any of claims 9-11, wherein the optical means comprises a dark-field microscope.

13. Method according to any of claims 9-12 wherein the image analysis is a software analysis of the spatially and spectrally resolved images taken with the optical means:
a) reading data of the acquired images from storage means;
b) correcting the read data to reduce inhomogeneities and noise of the acquired images;
c) localizing particles in the acquired images using the corrected data to obtain a position for each particle;
d) characterizing each particle individually to obtain an intermediate analysis result which comprises the position and characterization parameters for each particle;
e) classifying the particles based on the characterization parameters of each particle to obtain classes of particles;
f) counting a number of particles per class for each acquired image;
g) calculating an overall analysis result which comprises at least one statistical value, which is calculated for each biomarker in each sample of the biosensor using the number of particles per class for all the images acquired from the same sample, wherein the at least one statistical value per sample is correlated with an indication of the presence of a biomarker in the sample.

14. A biosensing platform for simultaneous, multiplexed, high throughput and ultra-sensitive optical detection of biomarkers labelled with plasmonic nanoparticles comprising the biodetection system of any of claims 1-8 as biosensor (BS).

15. A biosensing platform according to claim 14 further comprising a broadband and continuous spectrum illumination system (IS), an optical detector (OD) for simultaneously capturing and resolving spatially and spectrally the scattering signal of each individual nanoparticle, an autofocus system (AF) and an optical system (OS) adapted to collect the scattered light from the biosensor's surface onto the optical detector (OD), the platform being provided with translation means (MS) for the movement of the optical system and/or the biosensor, such that the optical system (OS) and the biosensor (BS) can be displaced relative to each other in the three dimensions, and wherein the processing means are adapted to:
iii) simultaneously capture spatially and spectrally resolved scattering signals from each nanoparticle individually, and
iv) to analyze these signals simultaneously with the capture process.

16. A biosensing platform according to claim 14 wherein:
a) the light source of the illumination system (IS) is a tungsten, halogen, xenon lamp or a continuum laser or a combination of multiple LEDs or LASERs in the visible and near-infrared spectral range; and/or
b) the optical system (OS) includes a microscope objective; and/or
c) the optical system (OS) includes an infinity-corrected microscope objective combined with a tube lens; and/or
d) the auto focus system (AF) is based on programming means.
